# EUROPEAN PATENT SPECIFICATION

(11) **EP 0 931 090 B1**
(45) Date of publication and mention of the grant of the patent: **21.04.2004**
(21) Application number: 98908601.2
(22) Date of filing: 19.02.1998
(51) Int. Cl.: C07H 21/00, A61K 31/70

(54) **IMPROVED CHIMERIC OLIGONUCLEOTIDE VECTORS**
VERBESSERTE CHIMÄRE OLIGONUKLEOTIDVEKTOREN
VECTEURS OLIGONUCLEOTIDIQUES CHIMERES AMELIORES

(30) Priority: 03.03.1997 US 39244 P
(43) Date of publication of application: 28.07.1999
(73) Proprietor: Applera Corporation, Foster City, CA 94404 (US)
(72) Inventor: ANDRUS, Alex, San Francisco, CA 94127 (US); KUIMELIS, Robert, G., Brighton, MA 02135 (US)
(74) Representative: Vossius, Volker, Dr.
(86) International application number: PCT/US1998/003224
(87) International publication number: WO 1998/039353

(56) References cited:
- WO-A-95/15972
- US-A- 5 565 350
- KMIEC, ERIC B.: "Genetic manipulation in mammalian cells using an RNA/DNA chimeric oligonucleotide" ADV. DRUG DELIVERY REV., vol. 17, 1995, pages 333-40, XP000618760
- YOON, KYONGGEUN; COLE-STRAUSS, ALLYSON; KMIEC, ERIC B.: "Targeted gene correction of episomal DNA in mammalian cells mediated by a chimeric RNA.cntdot.DNA oligonucleotide" PROC. NATL. ACAD. SCI. U. S. A., vol. 93, 1996, pages 2071-6, XP002068332 cited in the application
- HOSONO, KAZUMI; TSUKAHARA, SATORU; TAKAI, KAZUYUKI; TAKAKU, HIROSHI: "Study of base-pairing in the stem region of hairpin antisense oligonucleotides containing 2'-methoxynucleosides" NUCLEIC ACIDS SYMP. SER. 31(21ST SYMPOSIUM ON NUCLEIC ACIDS CHEMISTRY, 1994), 1994, pages 67-8, XP002068333
- ANDRUS, ALEX; VINAYAK, RAVI; KUIMELIS, ROBERT G.; MULLAH,BASHAR: "Targeted gene correction: synthesis and characterization of double-hairpin 2'-O-methyl RNA/DNA chimera oligonucleotides " NUCLEOSIDES NUCLEOTIDES, vol. 16, no. 7-9, 1997, pages 1183-94, XP002068334
- UHLMANN E.; PEYMANN, A.: "Antisense oligonucleotides: a new therapeutic principle" CHEM. REV., vol. 90, 1990, pages 544-584, XP000141412

## Description

### FIELD OF THE INVENTION

This invention relates to chimeric oligonucleotides useful as agents to effect specific genetic alterations in a target nucleic acid located in a living organism.

### REFERENCES

Alul, R. and Hoke, G., *Antisense Research & Development*, 5:3-11 (1995)
Andrus etal, "Targeted gene correction: synthesis and characterization of double-hairpin 2'-O-methyl RNA/DNA chimera oligonucleotides", *Nucleosides & Nucleotides*, 16:1183-94 (1997)
Andrus, A. etal, U.S. Patent No. 5,047,524 (1991) Automated system for polynucleotide synthesis and purification
Andrus, A. etal, U.S. Patent No. 5,262,530 (1993) Automated system for polynucleotide synthesis and purification
Andrus, A., "Chemical methods for 5' non-isotopic labelling of PCR probes and primers" in *PCR 2: A Practical Approach,* (1995), Oxford University Press, Oxford, pp. 39-54.
Antao et al, *Nucleic Acids Research,* 19:5901 (1991)
Applied Biosystems, *Evaluating and Isolating Synthetic Oligonucleotides*, (1992) Appendix I, p. A- 1 to A-7
Applied Biosystems, Users Manual - Models 392 and 394 DNA/RNA Synthesizers, p. 6-1 - 6-22, Part No. 901237 (1991).
Beaucage and Iyer, *Tetrahedron,* 48:2223-2311 (1992)
Blackburn, M. and Gait, M. *Nucleic Acids in Chemistry and Biology* (1996) Oxford University Press, p. 217-221.
Breslauer, K.J etal, "Predicting DNA duplex stability from the base sequence", *Proc. Nat. Acad*. *Sci.,* 83:3746-3750 (1986)
Brinster etal., "Targeted correction of a major histocompatibility class II Eα gene by DNA microinjected into mouse eggs", *Proc. Natl*. *Acad. Sci. USA*, 86:7087 (1989)
Bronstein, I. and Voyta, J., U.S. Patent No. 4,931,223, (1990). Methods of using chemiluminescent 1,2-dioxetanes
Bronstein, I., Edwards, B. and Voyta, J. *J*. *Biolumin*. *Chemilumin*. 4:99-111 (1989)
Capecchi, "Altering the genome by homologous recombination", *Science,* 244:1288 (1989)
Caruthers, M. and Beaucage, S., U.S. Patent No. 4,415,732 (1983) Phosphoramidite compounds and processes
Caruthers, M. and Matteucci, M., U.S. Patent No. 4,458,066 (1984) Process for preparing polynucleotides
Chaix etal, *Bioorganic & Medicinal Chemistry Letters*, 6:827- (1996)
Cole-Strauss etal, "Correction of the mutation responsible for sickle cell anemia by an RNA-DNA oligonucleotide", *Science,* 273:1386-89 (1996)
Damha etal, *Tetrahedron Letters*, 26:4839-42 (1985)
de la Torre etal, *Tetrahedron Letters*, 35:2733-36 (1994)
Diekmann etal, *Proc. Natl*. *Acad Sci.,* 84:8257- (1987)
Dorman etal, *Tetrahedron*, 40:95-102 (1984)
Durand etal, *Nucleic Acids Research,* 18:6353- (1990)
Froehler, B. etal, *Biochemistry,* 31:1603-09 (1992)
Goodchild, J., *Nucleic Acids Research,* 20:4607-12 (1992)
Gryaznov, S. and Letsinger, R., *Nucleic Acids Research,* 20:3403- (1992)
Hermanson, G., *Bioconjugate Techniques,* Chapter 17, p. 639-71, Academic Press (1996)
Howley, P.M. etal, "A rapid method for detecting and mapping homology between heterologous DNAs", *J*. *Biol*. *Chem*., 254:4876-83 (1979)
Ivinov, V.I., Minchenikova, L.E., Schyolkina, A.K. and Poletayev, A.I. *Biopolymers,* 12:89-110 (1973).
Johnson, W.C. Jr, in *Methods of Biochemical Analysis*., Glick, D., Ed., (1985) John Wiley & Sons, New York, pp. 61-163
Kandimalla, E. and Agrawal, S. *Biochemistry*, 35:15332-39 (1996)
Kandimalla, E. etal, *Nucleic Acids Research,* 25:370-78 (1997)
Kmiec E., U.S. Patent No. 5,565,350, ( 1996) Compounds and Methods for Site Directed Mutations in Eukaryotic Cells
Kmiec, E., Cole-Strauss, A., Yoon, K. PCT WO 97/41141, Priority May 1, 1996, Methods and Compounds for Curing Diseases Caused by Mutations
Kornberg, A. *DNA Replication* (1980) Freeman, San Francisco
Kricka, L. *Nonisotopic DNA Probe Techniques*, (1992) Academic Press, San Diego, pp. 3-28
Lamond, A. etal, *Cell*, 58:383-90 (1989)
Lewin, B., *Genes V.* (1994) Wiley, New York, pp. 677-909.
Limbach etal, *Nucleic Acids Research,* 22:2183-96 (1994)
Loakes etal, *Nucleic Acids Research,* 22: 4039- (1994)
McCollum, C. and Andrus, A. *Tetrahedron Letters*, 32:4069-4072 (1991)
Morvan etal, *Nucleic Acids Research,* 14: 5019- (1986)
Newman, P. etal, *Biochemistry*, 29: 9891-9901 (1990)
Nichols etal, *Nature*, 369:492- (1994)
Nielsen, J. etal, *Science,* 254:1497 (1991)
Olsen etal, *J*. *Biol*. *Chem.* 271:7435-39 (1996)
Perbost etal, *Biochem. Biophys. Res*. *Comm.,* 165:742- (1989)
Pieken, W. etal, *Science,* 253:314 (1991)
Radman, M. and Wagner, R., "The high fidelity of DNA replication" *Science* 259:24-31 (1988)
Saenger, W., *Principles of Nucleic Acid Structure*. (1984) Springer-Verlag, New York.
Sambrook, J., Fritsch, E.F., Maniatis, T., Eds., *Molecular Cloning, A Laboratory Manual, 2nd Ed., Volume II,* Cold Spring Harbor, p. 11.46, (1989).
Schaap, A., U.S. Patent No. 4,962,192, (1990) Chemiluminescent 1,2-dioxetane compounds
Seela, F. and Driller, *Nucleic Acids Research,* 13: 911- (1985)
Seela, F. and Kehne, A., *Biochemistry*, 24: 7556-61 (1985)
Sharanabasava etal, *Journal of Organic Chemistry*, 62:523- (1997)
Sproat, B. in *Protocols for Oligonucleotides and Analogs* (1994) "Synthesis of 2'-O-alkyl oligoribonucleotides", Agrawal, S., Ed., Humana Press
Stryer, L., *Annu. Rev. Biochem*., 47:819-46 (1978)
Stryer, L., *Biochemistry Second Edition*, Chapter 22, p. 511-38, W.H. Freeman (1981)
Sun etal, *PNAS*, 86:9198- (1989)
Sund etal, *Tetrahedron*, 52:12275- (1996)
Switzer, C. etal, *Biochemistry*, 32:10489-96 (1993)
Wagner, T. etal, *Science,* 260:1510- (1993)
Wagner, U.S. Patent No. 4,873,191, (1989) "Genetic transformation of zygotes", filed Aug. 18, 1986.
Wang, S. and Kool, E., *Biochemistry*, 34:4125-32 (1995)
Watson, J. etal, *Molecular Biology of the Gene* (1987) Benjamin Cummings, Menlo Park, pp. 621-73.
Whiteley et al., U.S. Patent No. 5,242,794 (1993) Detection of specific sequences in nucleic acids
Williams, D. etal, *Biochemistry,* 30:4001-09 (1991)
Yoon, K., etal, "Targeted gene correction of episomal DNA in mammalian cells mediated by a chimeric RNA•DNA oligonucleotide", *Proc. Natl*. *Acad*. *Sci. USA,* 93: 2071-2076 (1996)

### BACKGROUND

It has been recently discovered that certain DNA/RNA chimeric oligonucleotide molecules are effective as agents to cause specific alterations in a target nucleic acid sequence located in a living organism (Cole-Strauss; Kmiec; Yoon). Generally, the chimeric oligonucleotides consist of a single oligonucleotide strand that adopts a nicked, double-hairpin motif dictated by intramolecular Watson/Crick base-pairing of the nucleotides making up the oligonucleotide. The folded duplex is thought to induce targeted gene correction via homologous recombination and endogenous repair mechanism processes, the precise details of which have yet to be fully elucidated (Blackburn; Kornberg; Radman; Watson; Lewin). An unexpected discovery is the unusual and unpredicted stability of the double-hairpin motif, observed by thermal melting measurements. Such chimeric oligonucleotides may find use as gene therapy agents and as means to construct transgenic organisms.

### SUMMARY

The present invention is directed towards chimeric oligonucleotides having improved physicochemical and biological properties and methods of using such chimeric oligonucleotides.

It is an object of the present invention to provide chimeric oligonucleotides having increased intramolecular and intermolecular duplex stability as compared with known chimeric oligonucleotides.

It is another object of the present invention to provide chimeric oligonucleotides having structures which induce intramolecular and intermolecular A-type helix formation.

It is yet another object of the present invention to provide chimeric oligonucleotides having increased resistance to nuclease degradation as compared with known chimeric oligonucleotides.

It is an additional object of the present invention to provide chimeric oligonucleotides having increased chemical stability, resistance to hydrolysis and degradation, as compared with known chimeric oligonucleotides.

It is an additional object of the present invention to provide chimeric oligonucleotides having highly-stable, double-hairpin conformations, as measured by thermal melting measurements.

It is another object of the present invention to provide chimeric oligonucleotides having an increased rate of transport into a living cell as compared with known chimeric oligonucleotides.

It is yet another object of the present invention to provide chimeric oligonucleotides which provide signaling, labeling, and detection capability.

In a first aspect, the foregoing and other objects of the present invention are achieved by a chimeric oligonucleotide having a modified stem portion including one or more 2'-O-alkyl-ribonucleotides, 2'-O-allyl-ribonucleotides, 2'-allyl ribonucleotides, 2'-amino-ribonucleotides, 2'-halo-ribonucleotides, 2'-methoxyethyl-ribonucleotides, 2'-branching group-ribonucleotides, and 2'-O-branching group-ribonucleotides. In a particularly preferred embodiment, the chimera of the first aspect includes a stem portion including one or more 2'-O-methyl ribonucleotides.

In a second preferred embodiment of the first aspect, the modified stem portion includes one or more nucleotides having an α-anomeric nucleotide base or a branching group.

In a third preferred embodiment of the first aspect, the modified stem portion includes one or more carbocyclic-nucleotides.

In a fourth preferred embodiment of the first aspect, the modified stem portion includes one or more modified internucleotide linkages selected from the group consisting of 2'-5'-linkage, inverted 3'-3' linkage, inverted 5'-5' linkage, a methyl phosphonate, non-bridging, N-substituted phosphoramidate, alkylated phosphotriester branched structure, 3'-N-phosphoramidate, and peptide nucleic acid, PNA.

In a fifth preferred embodiment of the first aspect, the modified stem portion includes one or more nucleobase analogs. Such nucleobase analogs include 5-alkyl pyrimidine, 5,6-dihydrouridine, 5,6-dihydrothymine, 5-methyl-cytosine, 5-propynyl-uridine, 5-propynyl-cytidine, 2-thio-thymine, isocytosine, pseudouridine, isocytidine, 2-thio-uridine, 7-deaza-adenine, 7-deaza-guanine, 2-thio-guanine, 2-thio-adenine, 2-thio-7-deaza-adenine, isoguanine, 7-deaza-guanine, xanthine, 7-deaza-xanthine, hypoxanthine, 7-deaza-xanthine, 2,6-diaminopurine, 2,6 diamino-7-deaza purine, and universal bases.

The chimeric oligonucleotide of the present invention comprises one or more loop portions comprising non-nucleosidic polymers.

In one embodiment the chimeric oligonucleotide of the present invention has a modified 3'-end and/or modified 5'-end. In one preferred embodiment, the 3'-terminal nucleotide is a 2',3'-dideoxynucleotide. In a second preferred embodiment, the 5'-end of the chimera is modified by including a 5'-5'-linkage, a 2'-5'-linkage, or a reporter group, preferably a chromophore, fluorescent dye or chemiluminescent group or precursor.

In a further embodiment the chimeric oligonucleotide of the present invention is conjugated with a conjugation moiety. In one embodiment, the conjugation moiety is a moiety for increasing intermolecular duplex stability including minor groove binders and intercalators. In a second embodiment, the conjugation moiety is a moiety for increasing the detectability of the chimera (Hermanson).

In a second aspect, the present invention includes a method for introducing a specific alteration into a target sequence located in a living organism selected from the group consisting of a prokaryote, plant and virus. The method includes the steps of (i) introducing a chimeric oligonucleotide of the present invention into the living organism, and (ii) monitoring the specific alteration of the target sequence.

In a third aspect, the present invention includes a method of producing a non-human transgenic animal by introducing a specific alteration in a target sequence of said animal. The method comprises the steps of (i) introducing a chimeric oligonucleotide of the present invention into the pronucleus of a ovum or into an embryonic stem cell and (ii) monitoring the specific alteration of the target sequence.

In a fourth aspect, the present invention includes the use of a chimeric oligonucleotide of the present invention for the preparation of a pharmaceutical composition for introducing a specific alteration in a target sequence.

These and other objects, features, and advantages of the present invention will become better understood with reference to the following description, drawings, and appended claims.

### BRIEF DESCRIPTION OF THE DRAWINGS

- FIG. 1: Schematic structure of a chimeric oligonucleotide in the double-hairpin structural motif according to the present invention
- FIG. 2: 2' Modifications of the ribonucleotide groups in the stem portion of chimeric oligonucleotides of the present invention
- FIG. 3: α-Anomeric nucleotides and branching groups in the stem portion of chimeric oligonucleotides of the present invention
- FIG. 4: Carbocyclic nucleotides in the stem portion of a chimeric oligonucleotide of the present invention
- FIG. 5: Modified internucleotide linkage attachments in the stem portion of a chimeric oligonucleotide of the present invention
- FIG. 6: Internucleotide analogs in the stem portion of a chimeric oligonucleotide of the present invention
- FIG. 7: Pyrimidine nucleobase analogs in the stem portion of a chimeric oligonucleotide of the present invention
- FIG. 8: Purine nucleobase analogs in the stem portion of a chimeric oligonucleotide of the present invention
- FIG. 9: Non-nucleosidic polymer and "tetraloop" sequence polynucleotide loop portion of a chimeric oligonucleotide of the present invention (SEQ ID NO:15-17)
- FIG. 10: Absorbance vs. temperature melting curve for a 68nt chimera (top panel), and the corresponding first-derivative curve (bottom panel) (SEQ ID NO:17)
- FIG. 11: Two stage melting behavior of a double-hairpin chimeric oligonucleotide (SEQ ID NO:17)
- FIG. 12: A series of chimeras. Arrows indicate a nick in the bottom strand, with the 3'-terminus to the right of the arrow (SEQ ID NO:1-14)
- FIG. 13: Circular dichroism spectrum of a 68nt chimera (solid line) (SEQ ID NO:4) and the corresponding all-DNA sequence (dashed line) (SEQ ID NO: 11)
- note:: In Figures 9-13, Bold letters represent 2'-OMe ribonucleosides. T letters in bold are 2'-OMe uridine ribonucleosides, U. Non-bolded letters are 2'-deoxynucleosides (DNA). PEO represents pentaethylene oxide.

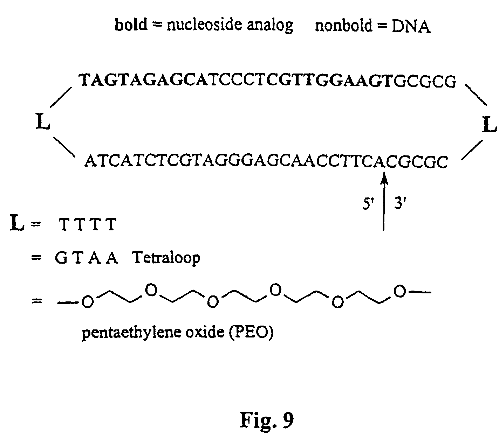

Oligonucleotides comprising nucleosidic loops as depicted in Fig. 9-13 are not within the scope of the claimed invention.

### DETAILED DESCRIPTION OF THE PREFERRED EMBODIMENTS

Reference will now be made in detail to certain preferred embodiments of the present invention, examples of which are illustrated in the accompanying drawings.

### 1. DEFINITIONS

Unless stated otherwise, the following terms and phrases as used herein are intended to have the following meanings:

The term "chimeric oligonucleotide" or "chimera" as used herein refers to an oligonucleotide including deoxyribonucleotide, ribonucleotide, and nucleoside analog monomers. The monomers are linked through phosphodiester and phosphodiester analog linkages. Examples of deoxyribonucleotide monomers include 2'-deoxyadenosine, 2'-deoxyguanosine, 2'-deoxycytidine, and thymidine. Examples of ribonucleotide monomers include adenosine, guanosine, cytidine, and uridine. Chimeras typically range in size from about 40-200 monomers in length.

The term "nucleotide analog" refers to analogs of natural deoxyribonucleotide and ribonucleotide monomers which include modified sugar and/or nucleobase portions, including but not limited to 2'-O-alkyl ribonucleotide, 2'-O-methoxy ribonucleotide, 2'-O-allyl ribonucleotide, 2'-halo ribonucleotide, 2-thiopyrimidine, C-5-propyne pyrimidine, 7-deazapurine, isocytosine, nucleosides containing an α-anomeric base, and carbocyclic-nucleotide.

The term "Watson/Crick base-pairing" refers to the pattern of specific pairs of nucleotides, and analogs thereof, that bind together through hydrogen-bonds, e.g. adenine (A) pairs with thymine (T) or uracil (U), and guanine (G) pairs with cytosine (C).

The term "phosphodiester analog" refers to analogs of natural, 3'-5' phosphodiester internucleotide linkages differing in their composition and/or location of attachment to a nucleotide, including but not limited to 2'-5'-linkage, 3'-3'-linkage, 5'-5'-linkage, methyl phosphonate, alkylated phosphotriester, 3'-N-phosphoramidate, and PNA.

The term "label attachment site" refers to the moiety on a nucleotide that connects said nucleotide to a label. Attachment sites can include the sugar, internucleotide, or nucleobase portion of the chimeric oligonucleotide.

The term "label" refers to a group attached to a nucleotide, nucleotide analog, or non-nucleosidic portion of a chimera. The label is capable of conducting a function such as giving a signal for detection of the molecule by such means as fluorescence, chemiluminescence, and electrochemical luminescence (Kricka). Alternatively, the label allows for separation or immobilization of the molecule by a specific or nonspecific capture method, e.g. biotin/avidin, antibody/ligand, and the like (Hermanson).

The term "branching group" refers to a pendant oligonucleotide moiety attached to a chimeric oligonucleotide such that the chimeric oligonucleotide contains more than one 3' or 5' terminus. Attachment sites can include the sugar, internucleotide, or nucleobase portion of the chimeric oligonucleotide.

The term "stem" refers to an intramolecular Watson/Crick base-pairing portion of a chimera whereby a stable, double-stranded, self-complementary conformation is evident from the sequence. A stem consists of contiguous nucleotides or nucleotide analogs base-paired with contiguous, complementary nucleotides or nucleotide analogs in a chimera.

The term "loop" refers to a single-stranded, non-complementary portion of a chimera consisting of nucleotides, nucleotide analogs, or a non-nucleoside polymer.

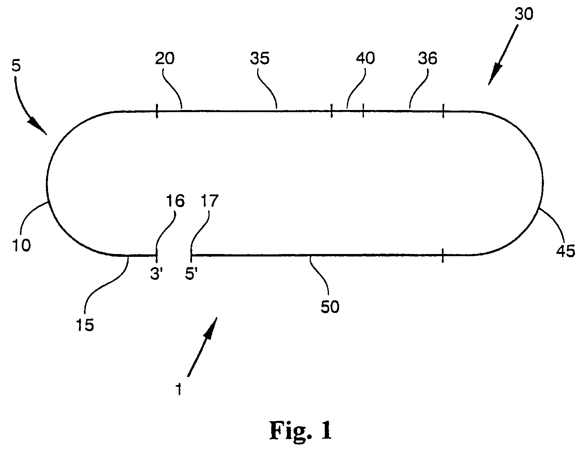

The term "double-hairpin" refers to the conformation of a chimera with two stem portions separated by a "nick", i.e., a site where the 3' and the 5' ends of the chimera are adjacent. The double-hairpin structural motif of chimera is characterized by stabilized thermal melting due to a high degree of self-complementarity and an affinity of nucleotide analogs in the stem portion. See Figure 1.

The term "lower alkyl" means linear or branched alkyl groups having from 1 to 8 carbon atoms, e.g., methyl, ethyl, propyl, isopropyl, tert-butyl, isobutyl, sec-butyl, neopentyl, tert-pentyl, and the like.

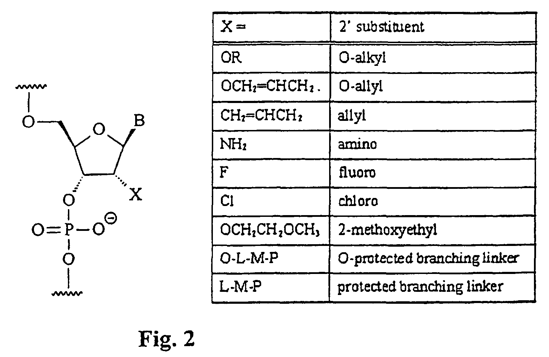

The term "2'-O-alkyl ribonucleotide" means a ribonucleotide in which a 2'-position on a sugar consists of the moiety ―OR where R is lower alkyl (Sproat 1994). The term 2'-O-methoxyethyl-ribonucleotide means a ribonucleotide in which a 2'-position on a sugar consists of a methoxyethyl moiety linked to the sugar through an oxygen atom. The term 2'-O-allyl-ribonucleotide means a ribonucleotide in which a 2'-position on a sugar consists of an allyl moiety linked to a sugar through an oxygen atom. The term 2'-allyl-ribonucleotide means a ribonucleotide in which a 2'-position on a sugar consists of an allyl moiety linked directly by a C-C bond to a 2'-carbon atom. The term 2'-halo-ribonucleotide means a ribonucleotide in which a 2'-position on a sugar consists of a halogen atom, e.g., F, and Cl (Williams). The term 2'-O-branching group-ribonucleotide means a ribonucleotide in which a 2'-position on a sugar consists of a branching group linked to the sugar through an oxygen atom (Damha). The term 2'- branching group-ribonucleotide means a ribonucleotide in which a 2'-position on a sugar consists of a branching group linked to a sugar directly through a 2'-carbon atom. The term 2'-amino ribonucleotide means a ribonucleotide in which a 2'-position on a sugar consists of an amino group linked to a sugar directly through a 2'-carbon atom. See Figure 2.

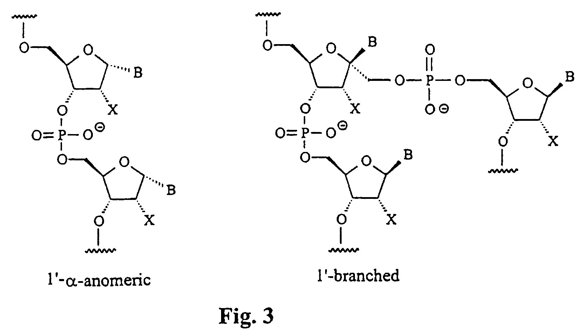

The term "α-anomeric base" refers to a nucleotide in which the natural sterochemistry of the 1'-position of the sugar is inverted, i.e., the heterocycle and 5'-atom are in a trans orientation instead of a cis orientation (Morvan). See Figure 3.

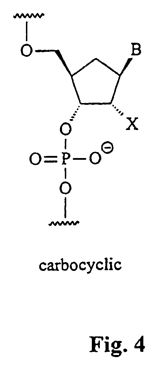

The term "carbocyclic-nucleotide" refers to a nucleotide in which the 4'-oxygen atom of the sugar is replaced with a carbon, sulfur, or nitrogen atom (Perbost). See Figure 4.

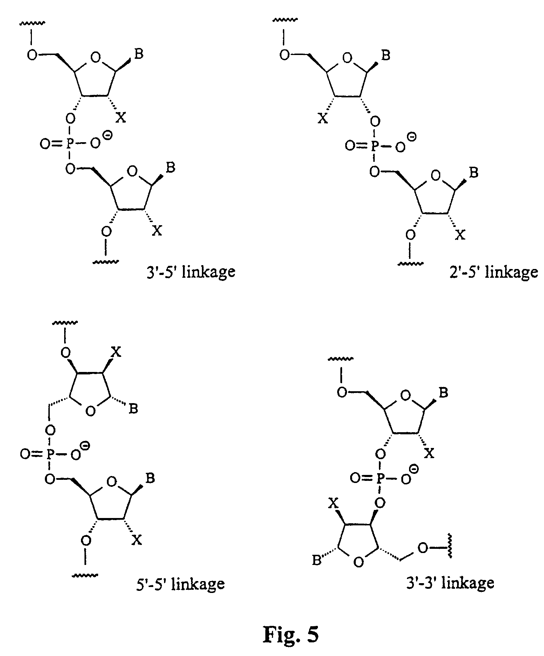

The term "2'-5'-linkage" refers to an internucleotide linkage in which a 2'-position of a first nucleotide is connected to a 5'-position of a second nucleotide through a phosphodiester linkage or analog thereof (Kandimalla; Alul). See Figure 5.

The term "inverted 3'-3' linkage" refers to an internucleotide linkage in which a 3'-position of a first nucleotide is connected to a 3'-position of a second nucleotide through a phosphodiester linkage or analog thereof(Froehler; Chaix). See Figure 5.

The term "5'-5' linkage" refers to an internucleotide linkage in which a 5'-position of a first nucleotide is connected to a 5'-position of a second nucleotide (Kandirnalla and Agrawal). See Figure 5.

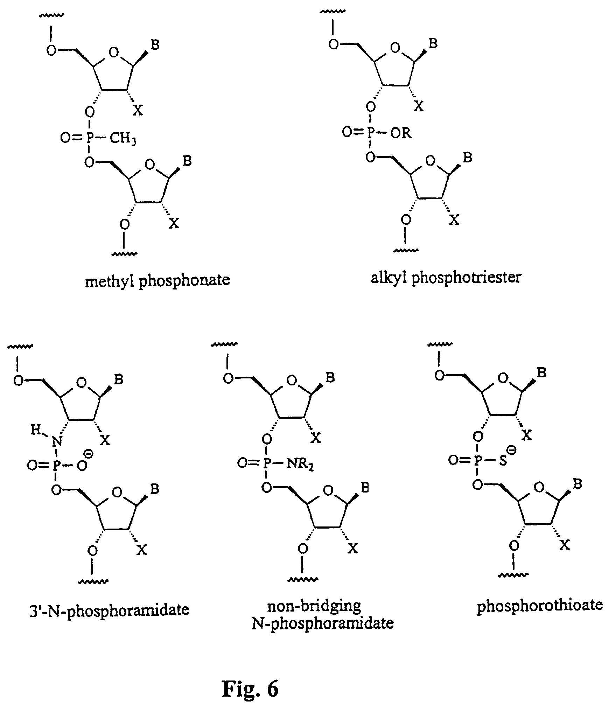

The term "methyl phosphonate" refers to a phosphate linkage analog in which one of the non-bridging oxygens is replaced with a methyl group (Dorman). See Figure 6.

The term "non-bridging N-substituted phosphoramidate" refers to a phosphate linkage analog in which one of the non-bridging oxygens is replaced with an ―NR₂ moiety where R is lower alkyl. See Figure 6.

The term "alkylated phosphotriester" refers to a phosphate linkage analog in which one of the non-bridging oxygens is bonded to an ―R moiety, where R is lower alkyl. See Figure 6.

The term "3'-N phosphoramidate" refers to a phosphate linkage analog in which an oxygen bonded to a 3'-position of a first nucleotide is replaced with ―NH― (Gryaznov). See Figure 6.

The term "peptide nucleic acid" or "PNA" refers to a structure in which nucleotide bases B are linked through hydroxyethyl glycine amide linkages rather than through ribose or deoxyribose sugars, e.g., the following structure (Nielsen).

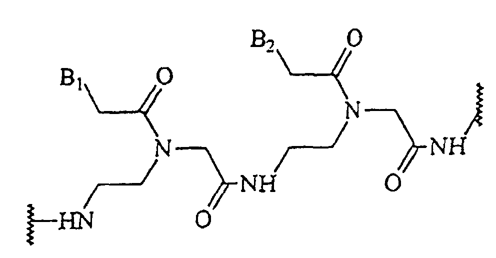

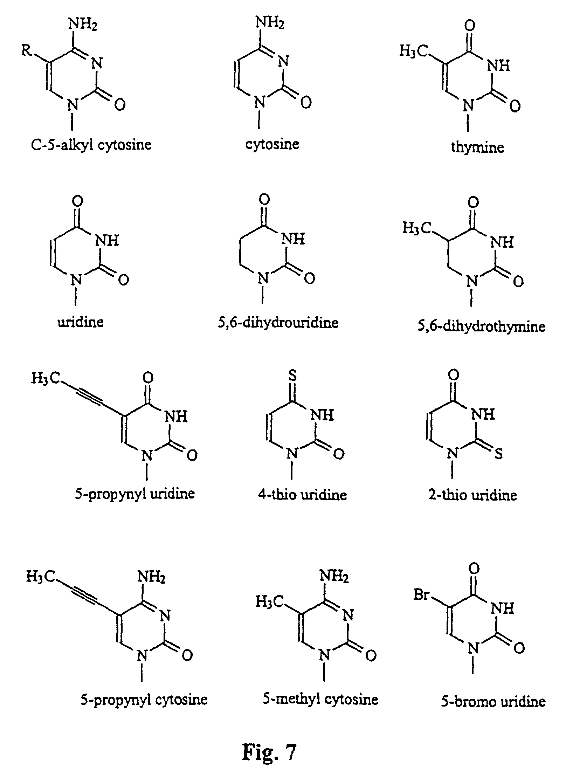

The term "C-5 alkylated pyrimidine" refers to a pyrimidine base having an alkyl group located at a C-5-position, e.g., 5-methyl-uracil and 5-methyl-cytosine (Wang). See Figure 7

The term "2-thiopyrimidine" refers to a pyrimidine base having a sulfur atom located at a 2-position, e.g., 2-thiouridine, 2-thiothymidine, and 2-thiocytidine (Newman). See Figure 7.

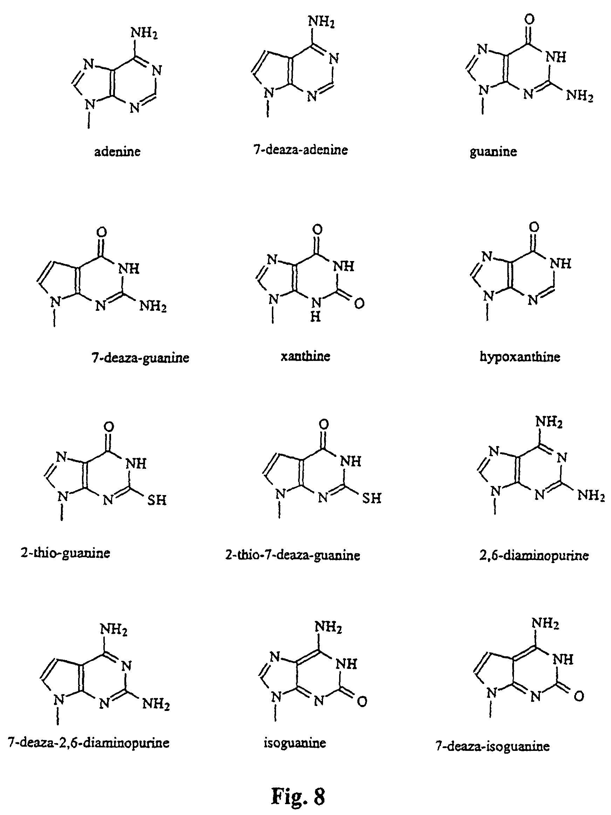

The term "isocytosine" refers to a cytosine base where a double-bonded oxygen is attached at a 4-position and an exocyclic amino is attached at a 2-position. Similarly, the term "isoguanine" refers to a guanine base where a double-bonded oxygen is attached at a 2-position and an exocyclic amino is attached at a 6-position (Switzer). See Figure 8.

The term "C-5-propyne pyrimidine" refers to a pyrimidine base having a propyne moiety located at the 5 position, where propyne is ―C≡C―, e.g., C-5 propyne cytidine and C-5 propyne thymidine (Wagner). See Figure 7.

The term "7-deaza purine" refers to a purine base wherein a nitrogen atom located at a 7-position is replaced by a carbon atom, e.g., 7-deaza-adenine and 7-deaza-guanine (Seela & Driller; Seela & Kehne). See Figure 8.

The term "2,6-diaminopurine" refers to a purine base having amino groups located at the 2- and 6-position, i.e., 2-amino adenosine (Diekmann). See Figure 8.

The term "universal base" refers to a nucleotide base which shows diminished base-specific discrimination in a Watson/Crick, base-pairing hybridization interaction, e.g., 3-nitropyrole (Nichols) and 5-nitroindole (Loakes).

The term "non-nucleosidic polymer" refers to a polymer which is not a polynucleotide, e.g., polyethylene oxide, polypeptide, polyacrylamide, and polycarbohydrate. See Figure 9.

The terms "tetranucleotide loop" and "tetraloop" refers to four-nucleotide sequences that form particularly stable, i.e., high melting temperature, hairpin loops (Antao). See Figure 9.

The term "nucleoside" refers to a compound consisting of a purine, deazapurine, or pyrimidine nucleoside base, e.g., adenine, guanine, cytosine, uracil, thymine, deazaadenine, deazaguanosine, and the like, linked to a pentose at the 1' position, including 2'-deoxy and 2'-hydroxyl forms (Stryer 1981). The term "nucleotide" as used herein refers to a phosphate ester of a nucleoside, e.g., mono-, di-, and triphosphate esters, wherein the most common site of esterification is a hydroxyl group attached at a C-5'-position of the pentose.

The term "chemiluminescent" refers to the light or photon generating capability of a compound. Typically chemiluminescence is initiated upon an event, such as cleavage of a bond, which generates an unstable intermediate that decomposes and releases light or a photon as part of the high-energy state decay process (Bronstein 1989; Bronstein 1990, Schaap 1990).

The term "detection" refers to detecting, observing, or measuring a nucleobase oligomer on the basis of the properties of a covalently-attached detection label. Detection labels include, but are not limited to, fluorescent dyes, e.g. fluorescein and rhodamine derivatives, cyanine dyes, energy-transfer dyes (Stryer 1978), radioisotopes, spin labels, and the like.

The term "capture" refers to capturing, immobilizing, separating, or sequestering a nucleobase oligomer on the basis of the properties of a capture label covalently attached to the nucleobase oligomer. Capture labels include, but are not limited to, biotin, digoxigenin, fluorescein, 2,4-dinitrophenyl, and hydrophobic modifiers such as cholesterol, trityl and trityl derivatives, polyethylene glycol, poly-lysine, triglycerides, and high-molecular weight hydrocarbons.

As used herein, the terms "polynucleotide" or "oligonucleotide" refer to linear, double and single stranded polymers of natural nucleotide monomers or analogs thereof, including deoxyribonucleotides, ribonucleotides, α-anomeric forms thereof, and the like. Usually the nucleoside monomers are linked by phosphodiester linkages, where as used herein, the term "phosphodiester linkage" refers to phosphodiester bonds or bonds including phosphate analogs thereof , including associated counterions, e.g., H⁺, NH₄⁺, Na⁺, and the like if such counterions are present. Polynucleotides typically range in size from a few monomeric units, e.g. 8-40, to several thousands of monomeric units.

"Template nucleic acid" refers to any nucleic acid capable of annealing with a chimera in a sequence specific manner. Exemplary template nucleic acids include DNA and RNA. More particularly, template nucleic acid may be genomic DNA, messenger RNA, cDNA, DNA amplification products from a PCR reaction, and the like.

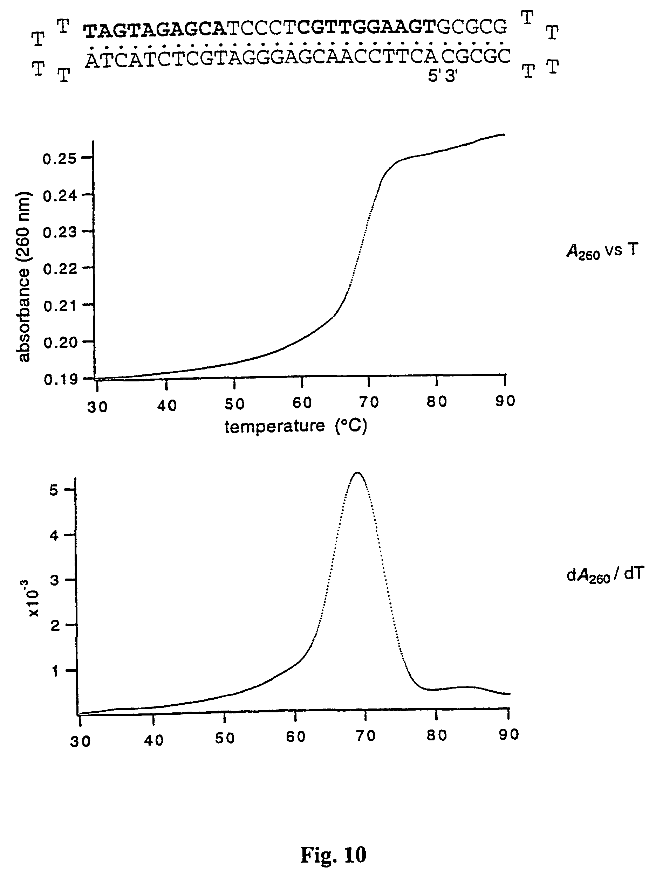

The term "thermal melting" refers to the disruption of hydrogen bonds and separation of strands of nucleic acid base-pairs in either an intramolecular or intermolecular construct. Thermal melting can be measured by a hyperchromic effect of increased UV absorbance when base-paired nucleic acids are heated (Wang). The temperature at which half of the base-pairs are disrupted is termed the "melting temperature" (Tm) and is measured by the inflection point, or first derivative, of a plot of temperature and absorbance (Figure 10).

### II. STRUCTURE OF THE CHIMERIC OLIGONUCLEOTIDES

Generally, referring to Figure 1, the chimeric oligonucleotide of the invention include a linear chimera **1** including first **35** and second **36** target complementary portions, a repair portion **40** located between the target complementary portions, and first **15**, second **20**, and third **50** stem portions. The first **35** and second **36** target complementary portions, repair portion **40**, and first **15**, second **20**, and third **50** stem portions will be referred to collectively herein as a "stem portion." The chimera further includes first **10** and second **45** loop portions, which will be referred to collectively herein as "loop portions." The chimera further includes a 3'-end **16** and a 5'-end **17**, which are not generally covalently joined but lie in close proximity when the chimera is in a hydrogen-bonded conformation.

Alternatively, the chimeras may have a single hairpin structure, i.e., regions **5**, **10**, **15**, and **20** are deleted. However, when the chimera is a single hairpin structure, because the 3'- and 5'-ends are no longer adjacent to each other, it is required that both the 3'- and 5'-ends of the chimera be modified so as to protect the ends from nuclease digestion.

Preferably, each of the first **35** and second **36** target complementary portions has a length of at least 4 nucleotides, preferably 8 or more nucleotides, and most preferably between about 20 to 30 nucleotides. Preferably, the combined length of the two target complementary portions is greater than 20 nucleotides, and more preferably between about 40 and 60 nucleotides. The sequence of the target complementary portions are complementary to a portion of the target sequence. It is thought that the endogenous repair enzymes separate the strands of both the chimera and the target and allow conventional duplex formation between the chimera and the target. In an important aspect of the present invention, a fraction of the nucleotides making up the target complementary portions are ribonucleotides or ribonucleotide analogs.

The repair portion **40** is interposed between the first **35** and second **36** target complementary portions and is between about 1 and 10 nucleotides in length. The sequence of the repair portion is not completely complementary to the target sequence. Rather the sequence of the repair portion includes at least one mismatch between the repair portion **40** and the target sequence. The sequence of the repair portion is substantially complementary to the portion of the target sequence which is to be altered. The nucleotides making up the repair portion are preferably deoxyribonucleotides.

While Figure 1 shows a chimeric oligonucleotides with a single repair sequence and two target complementary portions, the present invention includes chimeras having multiple repair portions and more than two target complementary portions.

The first **15**, second **20**, and third **50** stem portions serve to form a stable intramolecular duplex with the target complementary portions. Preferably, the first **15** and second **20** stem portions include a GC-clamp for stabilizing the duplex in the region of the first and second stem portions thereby protecting the chimera from enzymatic degradation, e.g., degradation by helicases and/or exonucleases. The first stem portion also includes a 3'-end **16** of the chimera.

The first **10** and second **45** loop portions serve to connect the second stem portion and target complementary portion to the first stem portion and third stem portion such that the chimera forms a stable intramolecular duplex structure. The loop portions preferably have a length of between 1 and 6 nucleotide equivalents, more preferably 4 nucleotide equivalents in length. The loop portions of the chimeric oligonucleotides of the present invention comprise non-nucleosidic polymers.

### A. Modified Stem Portion

Improved physiochemical and biological properties of chimeric vectors are achieved according to the present invention by modifying the chemical structure of the stem portion. Specifically, such modifications can impart a variety of advantageous features to the chimeric vectors of the invention including increased intramolecular and intermolecular duplex stability, increased intramolecular and intermolecular A-type helix formation, increased resistance to nuclease degradation, increased chemical stability, increased rate of transport into a living cell, and enhanced targeting and gene correction frequency.
*1. Modified Sugar Analogs*. In the chimeric oligonucleotides of the present invention a sugar portion of a fraction of the ribonucleotides making up a stem portion of a chimera is modified at the 2'-O-position of a nucleoside. Oligonucleotides bearing 2'-O-modified nucleosides have been studied as ribozymes, nuclease-resistance antisense analogs, and other cellular mechanism probes (Lamond etal; Olsen etal; Goodchild). Desirable features of 2'-O-alkyloligoribonucleosides include high chemical stability, substantial RNA- and DNA-nuclease resistance (including RNaseH), and increased thermal duplex stability. The 2'-O-methyl ribonucleosides are present in many types of RNA, and are formed by post-transcriptional processing in bacteria and eukaryotes (Limbach).
   In a preferred embodiment, a fraction of the ribonucleotides are 2'-O-alkyl-ribonucleotides, preferably 2'-O-methyl-ribonucleotides. Additional preferred modified ribonucleotides include 2'-O-allyl-ribonucleotides, 2'-allyl ribonucleotides, 2'-halo-ribonucleotides, 2'-O-methoxyethyl-ribonucleotides, 2'-branching group-ribonucleotides, and 2'-O-branching group-ribonucleotides (Figure 2).
   In a preferred embodiment of the invention, a 1'-position of a deoxyribonucleotide or ribonucleotide of the stem portion of the chimera is modified. In a preferred modification, the 1'-position includes an α-anomeric nucleotide base or a branching group (Figure 3).
   In another preferred modification of the nucleotides of the stem portion, the ribonucleotide or deoxyribonucleotide is a carbocyclic-nucleotide (Figure 4).
*2. Modified Internucleotide Linkages.* In another preferred embodiment of the chimeric oligonucleotides of the present invention, some or all of the nucleotides making up the stem portion of the chimera are linked through nonstandard internucleotide linkage analogs.
   Preferred nonstandard internucleotide linkages include 2'-5'-linkages, inverted 3'-3' and 5'-5' linkages (Figure 5), methyl phosphonate, non-bridging N-substituted phosphoramidate, alkylated phosphotriester branched structures, 3'-N-phosphoramidate (Figure 6), and peptide nucleic acid (PNA). More preferably, the linkages are PNA or 3'-N-phosphoramidate due to their high affinity.
*3. Nucleobase Analogs.* In yet another preferred embodiment of the chimeric oligonucleotides of the present invention, some or all of the nucleotides making up the stem portion of the chimera include nucleobase analogs (Figures 7 and 8). Preferred nucleobase analogs include C-5-alkyl pyrimidine, 2,6-diaminopurine, 2-thiopyrimidine, C-5-propyne pyrimidine, 7-deazapurine, isocytosine and isoguanine, and universal base. Preferably, the nucleobase analog is C-5-alkyl pyrimidine, C-5-propyne pyrimidine, or universal base.

### B. Modified Loops

Improved physicochemical and biological properties of chimeric vectors are further realized according to the present invention by including modified loop portions in the chimera. Specifically, such modifications provide for increased intramolecular duplex stability, increased resistance to nuclease degradation, and enhanced targeting and gene correction frequency.

The modified loop portions of the present invention include non-nucleosidic polymers rather than the traditional, non-complementary polynucleotide structures, e.g. poly-T. The non-nucleosidic polymers should be water soluble, available in well-characterized discrete sizes, chemically stable under physiological conditions, nuclease resistant, non-immunogenic, able to link to polynucleotides, and promote stable hairpin formation. Preferred non-nucleosidic polymers include polyethylene oxide, PEO (Durand), polymethylmethacrylate, polyacrylamide, polyvinyl alcohol, and the like. A particularly preferred non-nucleosidic polymer is polyethylene oxide having between 1 and 10 ethylene oxide (EO) units, e.g., triethylene oxide, pentaethylene oxide (PEO), and hexaethylene oxide.

The modified loops of the present invention may further include highly-stable hairpin structures that serve to improve the stability of the loop structures, e.g. sequences which form tetraloop structures (Figure 9).

### C. 5'- and 3'-End Modification

In a preferred embodiment of the present invention, improved physiochemical and biological properties of chimeric vectors are achieved by modifying the 5'- and 3'-ends of the chimera. Specifically, such modifications provide for increased resistance to nuclease degradation leading to enhanced targeting and gene correction frequency.

In a particularly preferred embodiment, a 3'-end of the chimera is modified by including a 2',3'-dideoxynucleotide as the 3'-end nucleotide. In another preferred embodiment, the 5'-end of the chimera is modified by including a 5'-5' linkage, a 2'-5' linkage, or a label, preferably a fluorescent dye or biotin.

### D. Chimera Conjugates

In a further preferred embodiment of the present invention, a chimera is conjugated with a label which may serve to increase intermolecular duplex stability, provide enhanced detectablity, and/or capturability. Preferred conjugates for increasing intermolecular duplex stability include minor groove binders (Sharanabasava), e.g., Hoechst 33258, intercalators (Sun), e.g., acridine, ethidium derivatives, and polycations, e.g., spermine (Sund).

To increase the detectability of the chimera, preferably the chimera is conjugated to a label, e.g. a fluorescent dye including fluorescein, rhodamine, cyanine, and the like. To facilitate capture or immobilization, a chimera may be conjugated with biotin or a peptide (de la Torre). The label may be attached to the chimera using any number of known conjugation methods (Andrus 1995; Hermanson).

### III. METHODS OF SYNTHESIS

Generally, the above modified chimeras are synthesized using known synthetic techniques. Detailed descriptions of the chemistry used to form polynucleotides are provided elsewhere (Beaucage; Applied Biosystems 1991). The phosphoramidite method of polynucleotide synthesis for making the chimeras of the invention is a preferred method because of its efficient and rapid coupling and the stability of the starting nucleoside monomers. The synthesis is typically performed with a growing polynucleotide chain attached to a solid support, so that excess reagents, which are in the liquid phase, can be easily removed by filtration, thereby eliminating the need for purification steps between cycles.

The following briefly describes the steps of a typical polynucleotide synthesis cycle using the phosphoramidite method. First, a solid support is treated with acid, e.g., trichloroacetic acid, to remove a hydroxyl protecting group, e.g., dimethoxytrityl group, freeing the hydroxyl for a subsequent coupling reaction. An activated intermediate is then formed by simultaneously adding a protected phosphoramidite nucleoside monomer or protected phosphoramidite loop portion, and a weak acid, e.g., tetrazole, and the like, to the reaction. The weak acid protonates a nitrogen of the phosphoramidite forming a reactive intermediate. Nucleoside addition is typically complete within 30 s. Next, a capping step is performed which terminates any polynucleotide chains that did not undergo nucleoside addition. Capping is preferably done with acetic anhydride and 1-methylimidazole. The internucleotide linkage is then converted from the phosphite to the more stable phosphotriester by oxidation using iodine as the preferred oxidizing agent and water as the oxygen donor. After oxidation, the hydroxyl protecting group is removed with a protic acid, e.g., trichloroacetic acid or dichloroacetic acid, and the cycle is repeated until chain elongation is complete. After synthesis, the polynucleotide chain is cleaved from the support using a base, e.g., ammonium hydroxide or t-butyl amine. The cleavage reaction also removes any phosphate protecting groups, e.g., cyanoethyl. Finally, the protecting groups on the exocyclic amines of the bases are removed by treating the polynucleotide solution in base at an elevated temperature, e.g., 55-65°C.

Synthesis efficiency is followed during the synthesis in real-time by measuring the detritylation effluent from the reaction column with a trityl conductivity monitor. Average stepwise yields are generally greater than 98%. High-cross link, 1000Å pore diameter polystyrene beads (McCollum; Andrus 1991; Andrus 1993), loaded at 12 µmole 3' nucleoside/gm support (PE Applied Biosystems, part # 401938) are used to generate about 40 crude odu (odu = absorbance at 260 nm of 1 ml volume in a 1 cm pathlength cell) of chimera at the 0.2 µmole scale (ca. 1.6 mg). Scale-up to a 1 µmole scale will give 200 crude odu (ca. 8 mg) using 1000Å, 3' nucleoside CPG support (PE Applied Biosystems, part # 401440). The nucleobase protecting groups are selected for comparable deprotection rates in concentrated ammonium hydroxide (1 hour at 65 °C) to minimize degradation or modification of the chimera oligonucleotide. After cleavage and deprotection, chimeras up to 80 bases in length are routinely attained in high purity and yield.

The synthesis of polynucleotides including nucleoside analogs, phosphodiester analogs, modified loop portions, chimera conjugates, or end modifications is described elsewhere. (See references associated with each modification set forth above.)

### IV. METHODS OF ANALYSIS AND PURIFICATION

Single-base resolution is challenging in the analysis and purification of chimeric oligonucleotides. The added complexity of multiple, stable conformations due to intramolecular hydrogen-bonding further complicates the task of purification of the chimera. For example, under the non-denaturing, reverse-phase conditions used in a conventional HPLC separation, multiple conformations are present, complicating product identification and collection. Slab polyacrylamide gel electrophoresis (PAGE) with 7 M urea as denaturant can be used for the analysis and purification of chimeras. Several odu (approx. 100 µg) can be isolated from an electrophoresis run by loading 10-20 crude odu on a 3 mm thick gel, performing the electrophoresis under standard conditions, excising the band after visualization under UV light against a TLC plate (EM Science, part # 5735), soaking in water overnight at room temperature, and desalting/concentrating on an oligonucleotide purification cartridge (PE Applied Biosystems, part # 400771). Anion-exchange HPLC on a polymeric adsorbent (Dionex NucleoPac PA-100; 4x250 mm, Dionex Co.) can give good resolution, predictable elution patterns, and reproducible retention times. A useful protocol entails the following: mobile phase - solvent A: 100 mM NaCl, 10 mM NaOH in 10 % acetonitrile (pH 12); solvent B: 800 mM NaCl, 10 mM NaOH in 10 % acetonitrile (pH 12); elution flow rate = 1.0 ml/min; and a linear gradient from 0% B at 0 min to 80% B at 25 min.

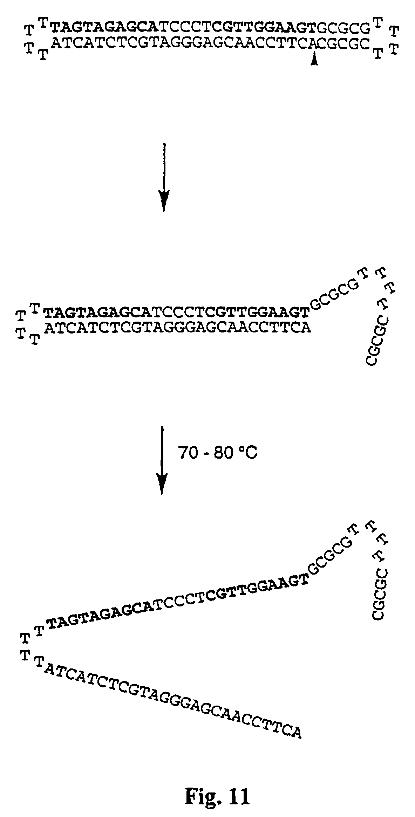

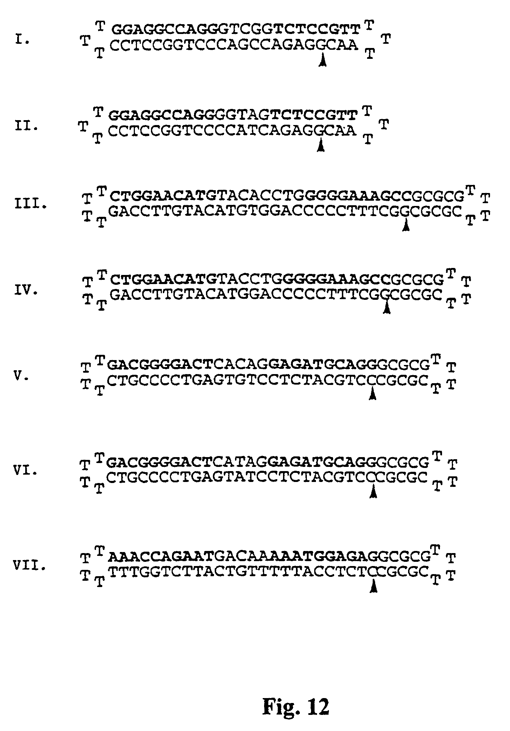

Thermal UV melting experiments can determine Tm values from the differentiated melting curves (Wang). Thermal melting curves of solutions of chimeric oligonucleotides typically exhibit a single, sharp transition consistent with a simple two-state model (Figure 10). The double-hairpin structural motif contains two domains, or helical regions, that melt independently of each other (Figure 11). The shorter helix (5 bp) closed by a TTTT hairpin loop melts near 70 °C in the indicated buffer system, whereas the longer helix (ca. 25 bp) closed by the same TTTT hairpin melts between 62-85 °C. The actual melting temperature is dictated by the sequence, base composition, and the modifications in the stem portion of the chimeric oligonucleotide. The 2'-OMe substitutions increase the thermal stability of the duplex by altering the helix structure (Figure 12). The chimera provides a 5.5-7.5 °C stabilization over the corresponding all-DNA sequence (Andrus 1997). The greater stability of the 2'-OMe/DNA heteroduplex over the corresponding DNA/DNA homoduplex may be critical for high-efficiency gene correction during the homologous recombination event, where the 2'-OMe regions must hybridize with the target sequence.

Unexpectedly, the currently available Tm prediction methods are not suitable for forecasting an accurate Tm of the chimeric oligonucleotides. An accurate predictive method must account for the thermodynamic contributions of the stem loops and the self-complementary nature of the sequence in addition to each individual nearest-neighbor interaction. Although this information is available for DNA and RNA sequences (Breslauer; Howley; Sambrook), the necessary data matrices have not yet been compiled for highly intramolecularly hydrogen-bonded nucleic acid analogs such as chimeric oligonucleotides. Table 1 compares the experimentally determined Tm values of the chimeras (Andrus 1997) from Figure 12 with three popular predictive methods.

**Table 1.**

| Comparison of measured and predicted melting temperatures (Tm) for a series of chimeras (Figure 12, SEQ ID NO:1-7) and the corresponding all-DNA sequences (Figure 12, SEQ ID NO:8-14). Predicted Tm values assume 25 mM sodium, 1 µM oligonucleotide, and do not include the non-base paired TTTT hairpin loops or the 5 bp region that melts independently. All values are in °C. | | | | | | |
|---|---|---|---|---|---|---|
| Chimera (Fig. 12) | all-DNA Measured | 2' O-Me Measured | ΔTm | Sambrook Predicted | Breslauer Predicted | Howley Predicted |
| I | 80.0 | 85.8 | +5.8 | 60.7 | 70.0 | 58.6 |
| II | 76.3 | 83.8 | +7.5 | 58.6 | 68.0 | 53.3 |
| III | 71.3 | 76.8 | +5.5 | 60.5 | 88.0 | 64.1 |
| IV | 71.0 | 77.4 | +6.4 | 59.3 | 82.0 | 61.8 |
| V | 73.5 | 80.8 | +7.3 | 61.1 | 82.0 | 62.5 |
| VI | 71.8 | 78.1 | +6.3 | 59.5 | 80.0 | 59.6 |
| VII | 62.4 | 69.2 | +6.8 | 49.7 | 68.0 | 51.6 |

### V. APPLICATION OF THE CHIMERIC OLIGONUCLEOTIDES

The chimeric vectors of the present invention find particular utility as agents for introducing a sequence alteration into a target sequence located in a living organism. The sequence of the target complementary portion of the chimera is chosen so as to be complementary to the target sequence and the sequence of the repair portions of the chimera is chosen so as to effect the desired sequence alteration. The high affinity of the nucleoside analogs in the target complementary portions 35 and 36 (Figure 1) for the target sequence during the homologous recombination event correlates directly with the efficiency of the desired sequence alteration. The affinity of chimeric oligonucleotides for target sequence is predicted by thermal melting measurements. High Tm values for chimeric oligonucleotides can predict efficient targeted gene correction.

The target sequence may be located in any living organism, including but not limited to prokaryote, eukaryote, plant, animal, and virus. The target sequence may be located in a nucleus of a cell, e.g., genomic DNA, or may be extranuclear nucleic acid, e.g., plasmid, mitrochondrial nucleic acid, and the like.

To cause a sequence alteration in a target sequence located in a living organism the chimera must be introduced into the living organism. Any method which effects such introduction may be used with the chimeras of the present invention including but not limited to electroporation, DEAE-dextran, calcium phosphate precipitation, liposome mediated-fusion, and direct microinjection of cells.

The optimal conditions to effect a desired sequence alteration in a target sequence may be empirically determined using any one of a number of methods capable of monitoring the alteration in the target sequence (Cole-Strauss; Kmiec; Yoon). Exemplary methods include but are not limited to sequencing of the target sequence, performing an oligonucleotide ligation assay (Whiteley), and allele-specific PCR.

In one embodiment of the invention, the chimeric vector can be used to construct transgenic animals. The chimeric vector can be introduced into the pronucleus of a ovum by direct injection (Brinster; Wagner). Alternatively, the chimeric vector can be introduced into an embryonic stem cell, chimeric animals can be produced by aggregation of the embryonic stem cell with normal blastocyst cells, and transgenic animals can be recovered as offspring of the chimeric animals (Capecchi).

### EXAMPLES

The invention will be further clarified by a consideration of the following examples, which are intended to be purely exemplary of the present invention and not to in any way limit its scope.

### EXAMPLE 1

### Synthesis of Pentaethylene Oxide (PEO) Phosphoramidite B

### Synthesis of 1-O-(4,4-dimethoxytrityl)-3,6,9,12-tetraoxapentadecanediol A

Pentaethylene glycol (25.0 g, 103mmol, Fluka Corporation) is dissolved in 200 ml dichloromethane (DCM) under an argon atmosphere. To this solution is added 31.5 g (93 mmol, Chem-Impex Co.) 4,4'-dimethoxytrityl chloride (DMT-Cl) dissolved in 100 ml DCM with 30 ml pyridine. The reaction mixture is stirred at room temperature for 2 hours and extracted with 200 ml of aqueous 5%NaHCO₃ and 200 ml water. The resulting organic layer is dried over MgSO₄, concentrated to an oil, and purified by column chromatography eluting with hexane/ethylacetate containing 0.5% triethylamine (20:80 to 80:20). Product-containing fractions yield 28.1 g (52 mmol, 50%) of 1-O-(4,4'-dimethoxytrityl)-3,6,9,12-tetraoxapentadecanediol **A** as a colorless oil after concentration and drying.

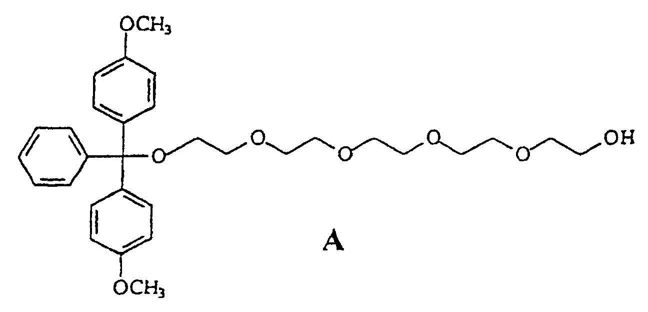

### Synthesis of 1-O-(4,4'-dimethoxytrityl)-14-O-[(N,N-diisopropylamino-O-2-cyanoethoxyphosphino]-3,6,9,12-tetraoxapentadecanediol B.

28.0 g 1-O-DMT-3,6,9,12-tetraoxapentadecanediol (52 mmol) A and 0.9 g of diisopropyl ammonium tetrazolide (5.2 mmol) are dissolved in 500 ml DCM. To this solution is added 17.2 g of 2-cyanoethoxy-(bis-N,N-diisopropylamino)-phosphine (57.0 mmol). The mixture is stirred 16 h at room temperature, at which time it is concentrated by evaporation to an oil. The crude oil is purified by redissolving it in 200 ml DCM and passing through a column containing 30 g alumina-B (Woelm Pharma). The DCM is removed and the subsequent product is dried under high vacuum to yield 34.0 g (44 mmol, 85%) of 1-O-(4,4'-dimethoxytrityl)-14-O-[(N,N-diisopropylamino-O-2-cyanoethoxyphosphino]-3,6,9,12-tetraoxapentadecanediol **B** as a light yellow oil.

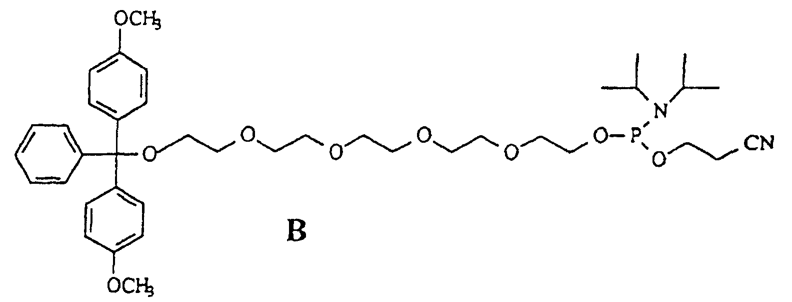

### EXAMPLE 2

### Synthesis of 2'-OMe Chimera and 2'-OMe Chimera Containing Non-Nucleosidic PEO Linkers

Chimera oligonucleotides are synthesized on an ABI 394 DNA/RNA synthesizer (PE Applied Biosystems, Foster City, CA) with DNA and 2'-OMe RNA phosphoramidite nucleoside monomers. The DNA monomers (part # 401183, 400326, 400328, 400329) are obtained from PE Applied Biosystems (Foster City, CA) and the 2'-OMe RNA monomers were obtained from Glen Research (Sterling, VA). The nucleobase protecting groups are benzoyl (A and C) and dimethylformamidine (G) for both the DNA and 2'-OMe RNA nucleosides. The non-nucleosidic PEO linker is also incorporated as a phosphoramidite synthon **B**, the synthesis of which is described above in Example 1. For each coupling cycle of the synthesis at the 0.2 µmole scale, 40 µl of 0.1 M phosphoramidite nucleoside (ca. 3.5 mg) in acetonitrile is delivered concurrently with 120 µl of 0.5 M 5-H tetrazole in acetonitrile. Coupling times are 25 seconds for DNA nucleosides and 4 minutes for 2'-OMe RNA nucleosides and the PEO synthon **B**.

The 60nt chimera is synthesized, as in Figure 9, where bold letters represent 2'-OMe ribonucleotide positions and (PEO) represents pentaethylene oxide (SEQ ID NO:15-17). The T bases in the bold 2'-OMe ribonucleotide positions are actually 2'-OMe uridine, U (Figures 9-13). Oligonucleotide chain assembly is performed in the 3' to 5' direction. In the case of the PEO-containing chimera, two different DNA/RNA synthesizers are necessary to accommodate the nine different monomers required because each instrument only has eight monomer bottle positions. After the first four DNA monomers are added to the solid support, the synthesis cartridge is removed from the first synthesizer and transferred to the second synthesizer, where the PEO coupling is performed. The synthesis cartridge is then returned to the original instrument and five additional DNA monomers are added, followed by ten 2'-OMe RNA monomers, six DNA monomers, and ten 2'-OMe RNA monomers, in that order. The synthesis cartridge is again transferred from the first to the second synthesizer, an additional PEO coupling is performed, and the cartridge is returned to the original instrument where 26 additional DNA couplings are performed.

### EXAMPLE 3

### Characterization of chimeric oligonucleotides by thermal melting

Thermal UV melting experiments can be performed on three chimeras to determine the intramolecular Tm values in both modified and unmodified chimeras. The structure of each of the three chimeras is as follows: Chimera 1 (SEQ BD NO:15) has the same structure as in Figure 9 (L = PEO); Chimera 2 (SEQ ID NO:18) has the same sequence as Chimera 1 except all the monomers are DNA and each of the PEO loop portions are replaced with four T nucleotides (L = TTTT); and Chimera 3 (SEQ ID NO:17) has the same sequence as Chimera 1 except each of the PEO loop portions is replaced with four T nucleotides (L = TTTT). The experiments are conducted in buffer containing 10 mM HEPES, pH 7.3, and 25 mM NaCl. Absorbance at 260 nm is monitored as a function of temperature between 30-90 °C at a heating rate of 0.5 °C min ⁻¹ on a Perkin-Elmer Lambda 12 spectrometer equipped with a PC-controlled Peltier heating unit. The Tm values for Chimeras 1, 2, and 3 are 77.2 °C, 71.0 °C, and 77.4 °C, respectively. The Tm difference between Chimera 2 and Chimera 3 (cf. 71.0 °C and 77.4 °C) reflects the extra stability that is afforded by inclusion of the 2'-OMe nucleotides. The Tm similarity between the Chimera 3 and Chimera 1 (cf. 77.4 °C and 77.2 °C) reflects the ability of a PEO linker to successfully form a hairpin loop without destabilizing the rest of the duplex.

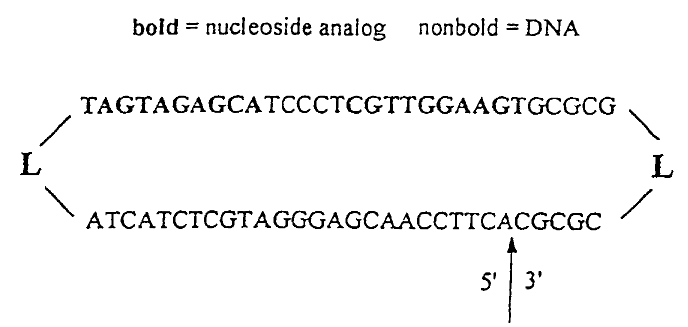

| Chimera | Loop L | nucleotides | Tm °C |
|---|---|---|---|
| 1 | PEO | 2'O-methyl RNA/DNA | 77.2 |
| 2 | TTTT | all DNA | 71.0 |
| 3 | TTTT | 2'O-methyl RNA/DNA | 77.4 |

### EXAMPLE 4

### Characterization of chimeric oligonucleotides by enzymatic digestion and HPLC analysis

Chimera oligonucleotides are checked for incomplete deprotection, base modifications, and correct base composition by enzymatic digestion with a mixture of snake venom phosphodiesterase and bacterial alkaline phosphatase and subsequent HPLC separation of the constituent nucleosides. All 8 nucleosides can be resolved and assigned by comparison with authentic samples upon reverse-phase HPLC analysis. The quantified base composition of the chimeras is in close agreement (<10% sum error) with the sequence using known and estimated extinction coefficients for each of the monomer units (Applied Biosystems 1992; Saenger). There are no detectable base modifications or residual protected deoxynucleosides detected.

Nucleoside composition of the chimera was assessed by digestion of the chimera (0.8 odu) with snake venom phosphodiesterase (0.1 U), bacterial alkaline phosphatase (0.7 U), 15mM MgCl₂, 30mM Tris, pH 7.5 at 37°C for 12 hours. The HPLC samples were ethanol precipitated twice, the supernatant dried thoroughly, and dissolved in H₂O. A useful HPLC protocol is: column - Applied Biosystems Spheri-5™ C18, 4.1x250 mm; mobile phase - A: 3% CH₃CN in 0.1 M TEAA, B: 90% CH₃CN in H₂O; flow rate - 0.5 ml/min; gradient - 0% B at 10 min.; 10% B at 35 min.; 100% Bat 60 min.

### EXAMPLE 5

### Characterization of chimeric oligonucleotides by circular dichroism and comparison with DNA

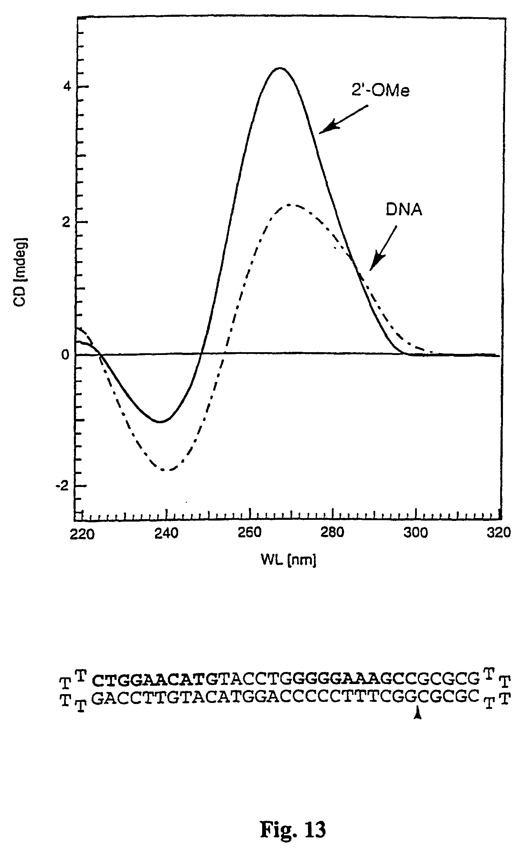

Circular dichroism is used to evaluate the conformation of the double-hairpin motif of the chimeric oligonucleotides (Johnson; Ivinov). The CD spectrum of the all-DNA sequence (SEQ ID NO:11) exhibits features that are consistent with a B-type helix; namely, equal intensity positive and negative peaks at 270 and 240 nm, respectively (Figure 13). In contrast, the spectrum of the 2'-OMe chimera (SEQ ID NO:4) shows features consistent with an A-type helix. It is well known that DNA/RNA heteroduplexes adopt an A-type helical structure due to the conformational restrictions imposed upon the carbohydrate by the 2'-OH group; the 2'-OMe substituent would impose similar restrictions (Saenger). Spectra were obtained with a Jasco J-600 spectrapolarimeter. Measurements were made with a 2 mm pathlength cell at 25 °C. Oligonucleotide samples were in 10 mM HEPES, pH 7.3, and 25 mM NaCl.

Although only a few embodiments have been described in detail above, those having ordinary skill in the art of genetic manipulation will clearly understand that many modifications are possible in the preferred embodiment without departing from the teachings thereof. All such modifications are intended to be encompassed within the following claims.

## Claims

1. A chimeric oligonucleotide comprising:
a self-complementary stem portion including one or more nucleotides selected from the group consisting of 2'-O-alkyl-ribonucleotides, 2'-O-allyl-ribonucleotides, 2'-allyl ribonucleotides, 2'-amino-ribonucleotides, 2'-halo-ribonucloeotides, 2'-O-methoxyethyl-ribonucleotides, 2'-branching group-ribonucleotides, and 2'-O-branching group-ribonucleotides;
one or more loop portions comprising non-nucleosidic polymers;
at least one 3'-end; and at least one 5'-end.

2. The chimeric oligonucleotide vector of claim 1 comprising a stem portion including one or more 2'-O-methyl ribonucleotides.

3. The chimeric oligonucleotide of claim 1 comprising a stem portion including one or more α-anomeric nucleotide bases or a branching group.

4. The chimeric oligonucleotide of claim 1 comprising a stem portion including one or more carbocyclic-nucleotides.

5. The chimeric oligonucleotide of claim 1 comprising a stem portion including one or more internucleotide linkages selected from the group consisting of 2'-5'-linkage, inverted 3'-3' linkage, inverted 5'-5' linkage, methyl phosphonate, non-bridging N-substituted phosphoramidate, alkylated phosphotriester branched structure, 3'-N-phosphoramidate, and peptide nucleic acid (PNA).

6. The chimeric oligonucleotide of claim 1 comprising a stem portion including one or more nucleotides including a nucleoside analog selected from the group consisting of C-5-alkyl pyrimidines, 2,6-diaminopurine, 2-thiopyrimidine, C-5-propyne pyrimidine, 7-deazapurine, isocytidine, isoguanosine, and universal base.

7. The chimeric oligonucleotide of claim 1 comprising a stem portion having a 3'-terminal nucleotide wherein the 3'-terminal nucleotide is a 2',3'-dideoxynucleotide.

8. The chimeric oligonucleotide of claim 1 further comprising labels selected from the group consisting of fluorescein, rhodamine, cyanine, biotin, dinitrophenyl, acridine, digoxigenin and peptides.

9. The chimeric oligonucleotide of claim 1 further comprising labels selected from chemiluminescent precursors having the structure 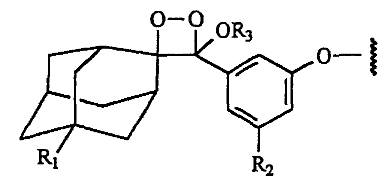 where R₁ is hydrogen or halogen; R₂ is phosphate, galactoside, glucoside, glucuronide, trialkylsilyloxy, acyloxy, or hydrogen; and R₃ is methyl, ethyl, and lower alkyl.

10. A method of introducing a specific alteration in a target sequence of a living organism selected from the group consisting of a prokaryote, plant and virus comprising the steps of:
introducing a chimeric oligonucleotide of any one of claims 1 to 9 into the living organism and
monitoring the specific alteration of the target sequence.

11. The method of claim 10, wherein the target sequence is located in a nucleus of a cell.

12. A method of producing a non-human transgenic animal by introducing a specific alteration in a target sequence of said animal comprising the steps of:
introducing a chimeric oligonucleotide of any one of claims 1 to 9 into the pronucleus of a ovum or into an embryonic stem cell and
monitoring the specific alteration of the target sequence.

13. The method of claim 12, wherein the target sequence is located in a nucleus of a cell.

14. Use of a chimeric oligonucleotide of any one of claims 1 to 9 for the preparation of a pharmaceutical composition for introducing a specific alteration in a target sequence.

15. The use of claim 14, wherein the target sequence is located in a nucleus of a cell.

## Patentansprüche

1. Chimäres Oligonukleotid, umfassend:
einen selbst-komplementären Stammanteil, einschließlich eines oder mehrerer Nukleotide, ausgewählt aus der Gruppe bestehend aus 2'-O-Alkylribonukleotiden, 2'-O-Allylribonukleotiden, 2'-Allylribonukleotiden, 2'-Aminoribonukleotiden, 2'-Halogenribonukleotiden, 2'-O-Methoxyethylribonukleotiden, 2'-Verzweigungsgruppe-Ribonukleotiden und 2'-O-Verzweigungsgruppe-Ribonukleotiden,
einen oder mehrere Schleifenanteile, die nicht-nukleosidische Polymere umfassen, mindestens ein 3'-Ende und mindestens ein 5'-Ende.

2. Chimäres Oligonukleotid nach Anspruch 1, umfassend einen Stammanteil, der ein oder mehrere 2'-O-Methylribonukleotide enthält.

3. Chimäres Oligonukleotid nach Anspruch 1, umfassend einen Stammanteil, der eine oder mehrere α-anomerische Nukleotidbasen oder eine Verzweigungsgruppe enthält.

4. Chimäres Oligonukleotid nach Anspruch 1, umfassend einen Stammanteil, der ein oder mehrere carbocyclische Nukleotide enthält.

5. Chimäres Oligonukleotid nach Anspruch 1, umfassend einen Stammanteil, der eine oder mehrere Internukleotid-Bindungen enthält, ausgewählt aus der Gruppe bestehend aus 2'-5'-Bindung, invertierter 3'-3'-Bindung, invertierter 5'-5'-Bindung, Methylphosphonat, nicht-verbrückendem N-substituiertem Phosphoramidat, alkylierter Phosphotriester-verzweigter Struktur, 3'-N-Phosphoramidat und Peptidnukleinsäure (PNA).

6. Chimäres Oligonukleotid nach Anspruch 1, umfassend einen Stammanteil, der eine oder mehrere Nukleotide enthält, einschließlich eines Nukleosid-Analogons, ausgewählt aus der Gruppe bestehend aus C-5-Alkylpyrimidinen, 2,6-Diaminopurin, 2-Thiopyrimidin, C-5-Propinpyrimidin, 7-Deazapurin, Isocytidin, Isoguanosin und Universalbase.

7. Chimäres Oligonukleotid nach Anspruch 1, umfassend einen Stammanteil mit einem 3'-terminalen Nukleotid, wobei das 3'-terminale Nukleotid ein 2',3'-Didesoxynukleotid ist.

8. Chimäres Oligonukleotid nach Anspruch 1, das ferner Markierungen umfasst, ausgewählt aus der Gruppe bestehend aus Fluorescein, Rhodamin, Cyanin, Biotin, Dinitrophenyl, Acridin, Digoxigenin und Peptiden.

9. Chimäres Oligonukleotid nach Anspruch 1, das ferner Markierungen umfasst, ausgewählt aus chemilumineszierenden Vorläufern mit der Struktur 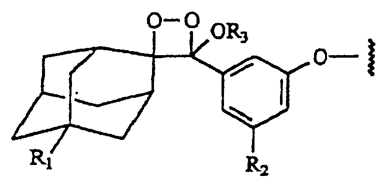 worin R₁ ein Wasserstoff- oder Halogenatom ist, R₂ eine Phosphat-, Galactosid-, Glucosid-, Glucuronid-, Trialkylsilyloxy-, Acyloxygruppe oder ein Wasserstoffatom ist und R₃ eine Methyl-, Ethyl- oder Niederalkylgruppe ist.

10. Verfahren zum Einbringen einer spezifischen Abänderung in einer Zielsequenz eines lebenden Organismus, ausgewählt aus der Gruppe bestehend aus einem Prokaryonten, einer Pflanze und einem Virus, umfassend die Schritte:
Einbringen eines chimären Oligonukleotids nach einem der Ansprüche 1 bis 9 in den lebenden Organismus und
Überwachen der spezifischen Abänderung der Zielsequenz.

11. Verfahren nach Anspruch 10, wobei die Zielsequenz in einem Kern einer Zelle lokalisiert ist.

12. Verfahren zum Herstellen eines nicht-menschlichen transgenen Tiers durch Einbringen einer spezifischen Abänderung in einer Zielsequenz des Tiers, umfassend die Schritte:
Einbringen eines chimären Oligonukleotids nach einem der Ansprüche 1 bis 9 in den Pronukleus einer Eizelle oder in eine embryonale Stammzelle und
Überwachen der spezifischen Abänderung der Zielsequenz.

13. Verfahren nach Anspruch 12, wobei die Zielsequenz in einem Kern einer Zelle lokalisiert ist.

14. Verwendung eines chimären Oligonukleotids nach einem der Ansprüche 1 bis 9 für die Herstellung einer pharmazeutischen Zusammensetzung zum Einbringen einer spezifischen Abänderung in einer Zielsequenz.

15. Verwendung nach Anspruch 14, wobei die Zielsequenz in einem Kern einer Zelle lokalisiert ist.

## Revendications

1. Oligonucléotide chimère comprenant:
une portion souche auto-complémentaire comportant un ou plusieurs nucléotides choisis dans le groupe consistant en 2'-O-alkyl-ribonucléotides, 2'-O-allyl-ribonucléotides, 2'-allyl-ribonucléotides, 2'-amino-ribonucléotides, 2'-halogéno-ribonucléotides, 2'-O-méthoxy-éthyl-ribonucléotides, 2'-groupe ramifiant-ribonucléotides, et 2'-O-groupe ramifiant-ribonucléotides;
une ou plusieurs portions de boucle comprenant des polymères non-nucléosidiques;
au moins une extrémité 3'; et au moins une extrémité 5'.

2. Vecteur oligonucléotidique chimère selon la revendication 1, comprenant une portion souche comportant un ou plusieurs 2'-O-méthylribonucléotides.

3. Oligonucléotide chimère selon la revendication 1, comprenant une portion souche comportant une ou plusieurs bases nucléotidiques α-anomères ou un groupe ramifiant.

4. Oligonucléotide chimère selon la revendication 1, comprenant une portion souche comportant un ou plusieurs nucléotides carboxyliques.

5. Oligonucléotide chimère selon la revendication 1, comprenant une portion souche comportant une ou plusieurs liaisons internucléotidiques choisies dans le groupe consistant en liaison 2'-5', liaison 3'-3' inversée, liaison 5'-5' inversée, méthyl phosphonate, phosphoramidate N-substitué non-pontant, structure ramifiée de phosphotriester alkylé, 3'-N-phosphoramidate, et acide nucléique peptidique (PNA).

6. Oligonucléotide chimère selon la revendication 1, comprenant une portion souche comportant un ou plusieurs nucléotides incluant un analogue nucléosidique choisi dans le groupe consistant en C-5-alkyl pyrimidines, 2,6-diaminopurine, 2-thiopyrimidine, C-5-propyne pyrimidine, 7-déazapurine, isocytidine, isoguanosine, et une base universelle.

7. Oligonucléotide chimère selon la revendication 1, comprenant une portion souche ayant un nucléotide 3'-terminal dans lequel le nucléotide 3'-terminal est un 2',3'-didésoxynucléotide.

8. Oligonucléotide chimère selon la revendication 1, comprenant en outre des marqueurs choisis dans le groupe consistant en fluorescéine, rhodamine, cyanine, biotine, dinitrophényle, acridine, digoxygénine et peptides.

9. Oligonucléotide chimère selon la revendication 1, comprenant en outre des marqueurs choisis parmi des précurseurs chimioluminescents ayant la structure 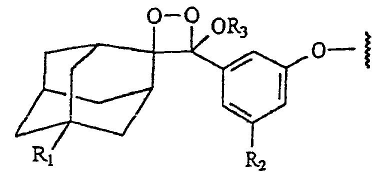 où R₁ est l'hydrogène ou un halogène; R₂ est un phosphate, galactoside, glucoside, glucuronide, trialkylsilyloxy, acyloxy, ou hydrogène; et R₃ est un méthyle, éthyle, et alkyle inférieur.

10. Procédé pour l'introduction d'une modification spécifique dans une séquence cible d'un organisme vivant choisi dans le groupe consistant en un procaryote, une plante et un virus, comprenant les étapes de:
introduction d'un oligonucléotide chimère selon l'une quelconque des revendications 1 à 9 dans l'organisme vivant, et
contrôle de la modification spécifique de la séquence cible.

11. Procédé selon la revendication 10, dans lequel la séquence cible est située dans un noyau d'une cellule.

12. Procédé pour la production d'un animal transgénique non-humain par introduction d'une modification spécifique dans une séquence cible du dit animal, comprenant les étapes de:
introduction d'un oligonucléotide chimère selon l'une quelconque des revendications 1 à 9 dans le pronucléus d'un ovule ou dans une cellule souche embryonnaire, et
contrôle de la modification spécifique de la séquence cible.

13. Procédé selon la revendication 12, dans lequel la séquence cible est située dans un noyau d'une cellule.

14. Utilisation d'un oligonucléotide chimère selon l'une quelconque des revendications 1 à 9 pour la préparation d'une composition pharmaceutique pour l'introduction d'une modification spécifique dans une séquence cible.

15. Utilisation selon la revendication 14, dans laquelle la séquence cible est située dans un noyau d'une cellule.


### SEQUENCE LISTING

(1) GENERAL INFORMATION
   (i) APPLICANT: Andrus, Alex Kuimelis, Robert G.
   (ii) TITLE OF THE INVENTION: IMPROVED CHIMERIC OLIGONUCLEOTIDE VECTORS
   (iii) NUMBER OF SEQUENCES: 18
   (iv) CORRESPONDENCE ADDRESS:
      (A) ADDRESSEE: The Perkin-Elmer corporation
      (B) STREET: 850 Lincoln Centre Drive
      (C) CITY: Foster City
      (D) STATE: California
      (E) COUNTRY: USA
      (F) ZIP: 94404
   (v) COMPUTER READABLE FORM:
      (A) MEDIUM TYPE: Diskette
      (B) COMPUTER: IBM Compatible
      (C) OPERATING SYSTEM: DOS
      (D) SOFTWARE: FastSEQ for Windows Version 2.0
   (vi) CURRENT APPLICATION DATA:
      (A) APPLICATION NUMBER: To Be Assigned
      (B) FILING DATE: 18-FEB-1998
   (viii) ATTORNEY/AGENT INFORMATION:
      (A) NAME: Bortner, Scott R.
      (B) REGISTRATION NUMBER: 34,298
      (C) REFERENCE/DOCKET NUMBER: 4319
   (ix) TELECOMMUNICATION INFORMATION:
      (A) TELEPHONE: (650) 638-6245
      (B) TELEFAX: (650) 638-6071
(2) INFORMATION FOR SEQ ID NO:1:
   (i) SEQUENCE CHARACTERISTICS:
      (A) LENGTH: 52 base pairs
      (B) TYPE: nucleic acid analog, 2'OMe RNA/DNA, am, cm, gm, um
      (C) STRANDEDNESS: single
      (D) TOPOLOGY: linear
   (ix) FEATURE: double-hairpin
   (xi) SEQUENCE DESCRIPTION: SEQ ID NO:1:
   GGAGACCGAC CCTGGCCTCC TTTNNNNNNN NNNGTCGGNN NNNNNNTTTA AC 52
(2) INFORMATION FOR SEQ ID NO:2:
   (i) SEQUENCE CHARACTERISTICS:
      (A) LENGTH: 52 base pairs
      (B) TYPE: nucleic acid analog, 2'OMe RNA/DNA, am, cm, gm, um
      (C) STRANDEDNESS: single
      (D) TOPOLOGY: linear
   (ix) FEATURE: double-hairpin
   (xi) SEQUENCE DESCRIPTION: SEQ ID NO:2:
   GGAGACTACC CCTGGCCTCC TTTNNNNNNN NNNGGTAGNN NNNNNNTTTA AC 52
(2) INFORMATION FOR SEQ ID NO:3:
   (i) SEQUENCE CHARACTERISTICS:
      (A) LENGTH: 74 base pairs
      (B) TYPE: nucleic acid analog, 2'OMe RNA/DNA, am, cm, gm, um
      (C) STRANDEDNESS: single
      (D) TOPOLOGY: linear
   (ix) FEATURE: double-hairpin
   (xi) SEQUENCE DESCRIPTION: SEQ ID NO:3:
   GGCTTTCCCC CAGGTGTACA TGTTCCAGTT TTNNNNNNNN NNTACACCTG NNNNNNNNNN 60
   GCGCGTTTTC GCGC 74
(2) INFORMATION FOR SEQ ID NO:4:
   (i) SEQUENCE CHARACTERISTICS:
      (A) LENGTH: 70 base pairs
      (B) TYPE: nucleic acid analog, 2'OMe RNA/DNA, am, cm, gm, um
      (C) STRANDEDNESS: single
      (D) TOPOLOGY: linear
   (ix) FEATURE: double-hairpin
   (xi) SEQUENCE DESCRIPTION: SEQ ID NO:4:
   GGCTTTCCCC CAGGTACATG TTCCAGTTTT NNNNNNNNNN TACCTGNNNN NNNNNNGCGC    60
   GTTTTCGCGC                                                           70
(2) INFORMATION FOR SEQ ID NO:5:
   (i) SEQUENCE CHARACTERISTICS:
      (A) LENGTH: 68 base pairs
      (B) TYPE: nucleic acid analog, 2'OMe RNA/DNA, am, cm, gm, um
      (C) STRANDEDNESS: single
      (D) TOPOLOGY: linear
   (ix) FEATURE: double-hairpin
   (xi) SEQUENCE DESCRIPTION: SEQ ID NO:5:
   CCTGCATCTC CTGTGAGTCC CCGTCTTTTN NNNNNNNNNC ACAGNNNNNN NNNNGCGCGT    60
   TTTCGCGC                                                             68
(2) INFORMATION FOR SEQ ID NO:6:
   (i) SEQUENCE CHARACTERISTICS:
      (A) LENGTH: 68 base pairs
      (B) TYPE: nucleic acid analog, 2'OMe RNA/DNA, am, cm, gm, um
      (C) STRANDEDNESS: single
      (D) TOPOLOGY: linear
   (ix) FEATURE: double-hairpin
   (xi) SEQUENCE DESCRIPTION: SEQ ID NO:6:
   CCTGCATCTC CTATGAGTCC CCGTCTTTTN NNNNNNNNNC ATAGNNNNNN NNNNGCGCGT    60
   TTTCGCGC                                                             68
(2) INFORMATION FOR SEQ ID NO:7:
   (i) SEQUENCE CHARACTERISTICS:
      (A) LENGTH: 68 base pairs
      (B) TYPE: nucleic acid analog, 2'OMe RNA/DNA, am, cm, gm, um
      (C) STRANDEDNESS: single
      (D) TOPOLOGY: linear
   (ix) FEATURE: double-hairpin
   (xi) SEQUENCE DESCRIPTION: SEQ ID NO:7:
   CTCTCCATTT TTGTCATTCT GGTTTTTTTN NNNNNNNNNG ACAANNNNNN NNNNGCGCGT 60
   TTTCGCGC 68
(2) INFORMATION FOR SEQ ID NO:8:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ix) FEATURE: double-hairpin
   (xi) SEQUENCE DESCRIPTION: SEQ ID NO:8:
   GGAGACCGAC CCTGGCCTCC TTTGGAGGCC AGGGTCGGTC TCCGTTTTTA AC 52
(2) INFORMATION FOR SEQ ID NO:9:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ix) FEATURE: double-hairpin
   (xi) SEQUENCE DESCRIPTION: SEQ ID NO:9:
   GGAGACTACC CCTGGCCTCC TTTGGAGGCC AGGGGTAGTC TCCGTTTTTA AC 52
(2) INFORMATION FOR SEQ ID NO:10:
   (i) SEQUENCE CHARACTERISTICS:
      (A) LENGTH: 74 base pairs
      (B) TYPE: nucleic acid
      (C) STRANDEDNESS: single
      (D) TOPOLOGY: linear
   (ix) FEATURE: double-hairpin
   (xi) SEQUENCE DESCRIPTION: SEQ ID NO:10:
   GGCTTTCCCC CAGGTGTACA TGTTCCAGTT TTCTGGAACA TGTACACCTG GGGGAAAGCC 60
   GCGCGTTTTC GCGC 74
(2) INFORMATION FOR SEQ ID NO:11:
   (i) SEQUENCE CHARACTERISTICS:
      (A) LENGTH: 70 base pairs
      (B) TYPE: nucleic acid
      (C) STRANDEDNESS: single
      (D) TOPOLOGY: linear
   (ix) FEATURE: double-hairpin
   (xi) SEQUENCE DESCRIPTION: SEQ ID NO:11:
   GGCTTTCCCC CAGGTACATG TTCCAGTTTT CTGGAACATG TACCTGGGGG AAAGCCGCGC    60
   GTTTTCGCGC                                                           70
(2) INFORMATION FOR SEQ ID NO:12:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ix) FEATURE: double-hairpin
   (xi) SEQUENCE DESCRIPTION: SEQ ID NO:12:
   CCTGCATCTC CTGTGAGTCC CCGTCTTTTG ACGGGGACTC ACAGGAGATG CAGGGCGCGT    60
   TTTCGCGC                                                             68
(2) INFORMATION FOR SEQ ID NO:13:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ix) FEATURE: double-hairpin
   (xi) SEQUENCE DESCRIPTION: SEQ ID NO:13:
   CCTGCATCTC CTATGAGTCC CCGTCTTTTG ACGGGGACTC ATAGGAGATG CAGGGCGCGT    60
   TTTCGCGC                                                             68
(2) INFORMATION FOR SEQ ID NO:14:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ix) FEATURE: double-hairpin
   (xi) SEQUENCE DESCRIPTION: SEQ ID NO:14:
   CTCTCCATTT TTGTCATTCT GGTTTTTTTA AACCAGAATG ACAAAAATGG AGAGGCGCGT    60
   TTTCGCGC                                                             68
(2) INFORMATION FOR SEQ ID NO:15:
   (i) SEQUENCE CHARACTERISTICS:
      (A) LENGTH: 68 base pairs
      (B) TYPE: nucleic acid analog, PEO/2'OMe RNA/DNA, am, cm, gm, um
      (C) STRANDEDNESS: single
      (D) TOPOLOGY: linear
   (ix) FEATURE: double-hairpin
   (xi) SEQUENCE DESCRIPTION: SEQ ID NO:15:
   ACTTCCAACG AGGGATGCTC TACTATTTTN NNNNNNNNNT CCCTNNNNNN NNNNGCGCGT    60
   TTTCGCGC                                                             68
(2) INFORMATION FOR SEQ ID NO:16:
   (i) SEQUENCE CHARACTERISTICS:
      (A) LENGTH: 68 base pairs
      (B) TYPE: nucleic acid 2'OMe RNA/DNA, am, cm, gm, um
      (C) STRANDEDNESS: single
      (D) TOPOLOGY: linear
   (ix) FEATURE: double-hairpin
   (xi) SEQUENCE DESCRIPTION: SEQ ID NO:16:
   ACTTCCAACG AGGGATGCTC TACTAGTAAN NNNNNNNNNT CCCTNNNNNN NNNNGCGCGG    60
   TAACGCGC                                                             68
(2) INFORMATION FOR SEQ ID NO:17:
   (i) SEQUENCE CHARACTERISTICS:
      (A) LENGTH: 62 base pairs
      (B) TYPE: nucleic acid 2'OMe RNA/DNA, am, cm, gm, um
      (C) STRANDEDNESS: single
      (D) TOPOLOGY: linear
   (ix) FEATURE: double-hairpin
   (xi) SEQUENCE DESCRIPTION: SEQ ID NO:17:
   ACTTCCAACG AGGGATGCTC TACTANNNNN NNNNNNTCCC TNNNNNNNNN NGCGCGNCGC 60
   GC 62
(2) INFORMATION FOR SEQ ID NO:18:
   (i) SEQUENCE CHARACTERISTICS:
      (A) LENGTH: 68 base pairs
      (B) TYPE: nucleic acid
      (C) STRANDEDNESS: single
      (D) TOPOLOGY: linear
   (ix) FEATURE: double-hairpin
   (xi) SEQUENCE DESCRIPTION: SEQ ID NO:18:
   ACTTCCAACG AGGGATGCTC TACTATTTTT AGTAGAGCAT CCCTCGTTGG AAGTGCGCGT 60
   TTTCGCGC 68